(19) Europäisches Patentamt
European Patent Office
Office européen des brevets

(11) Veröffentlichungsnummer: **0 014 847**
**A1**

(12) # EUROPÄISCHE PATENTANMELDUNG

(21) Anmeldenummer: **80100294.0**

(22) Anmeldetag: **22.01.80**

(51) Int. Cl.³: **C 07 D 231/12**
C 07 D 401/12, C 07 D 403/06
C 07 D 413/06, C 07 D 417/06
A 61 K 31/415, A 61 K 31/425
A 61 K 31/445, A 61 K 31/495
//C07D257/04, (C07D401/12, 231/12, 211/46), (C07D403/06, 231/12), (C07D413/06, 231/12, 263/56), (C07D417/06, 231/12, 277/64), (C07D417/06, 231/06, 231/12, 277/06)

(30) Priorität: **19.02.79 DE 2906252**

(43) Veröffentlichungstag der Anmeldung:
**03.09.80 Patentblatt 80/18**

(84) Benannte Vertragsstaaten:
**AT BE CH DE FR GB IT NL SE**

(71) Anmelder: **Merck Patent Gesellschaft mit beschränkter Haftung**
**Frankfurter Strasse 250**
**D-6100 Darmstadt(DE)**

(72) Erfinder: **Gante, Joachim, Dr.**
**Stormstrasse 4**
**D-6100 Darmstadt-Arheilgen(DE)**

(72) Erfinder: **Radunz, Hans-Eckart, Dr.**
**Hügelstrasse 13**
**D-6109 Mühltal-2(DE)**

(72) Erfinder: **Orth, Dieter, Dr.**
**Jahnstrasse 83**
**D-6100 Darmstadt(DE)**

(72) Erfinder: **Minck, Klaus, Dr.**
**Buchestrasse 8**
**D-6105 Ober-Ramstadt(DE)**

(72) Erfinder: **Wild, Albrecht, Dr.**
**Sonderbach 76**
**D-6148 Heppenheim(DE)**

(72) Erfinder: **Klockow, Michael, Dr.**
**Fuchsenhütte 42**
**D-6101 Rossdorf(DE)**

(54) **Pyrazolderivate, diese enthaltende pharmazeutische Zubereitungen und Verfahren zu ihrer Herstellung.**

(57) Pyrazolderivate der allgemeinen Formel I

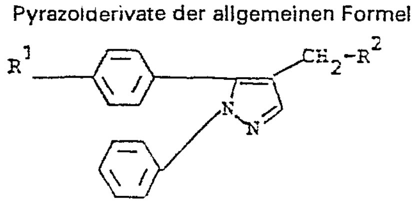

I

worin

$R^1$ H oder C1.

$R^2$ 1-Methyl-4-piperidyloxycarbonyl, 2-(4-Phenylpiperazino)-äthoxycarbonyl, Benzoxazol-2-yl, Benzothiazol- 2-yl, Tetrazol-5-yl oder 3-$R^3$-4-$R^4$-thiazolidin-2-yl,

$R^3$ H oder Acyl mit 1 - 7 C-Atomen und

$R^4$ H oder COOH

bedeuten

sowie ihre physiologisch unbedenkliche Salze wirken analgetisch und können hergestellt werden durch Reaktion einer Verbindung der allgemeinen Formel II

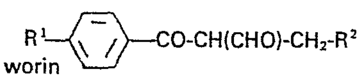

II

worin

, $R^1$ und $R^2$ die angegebene Bedeutung haben, oder eines ihrer reaktionsfähigen Derivate mit Phenylhydrazin.

EP 0 014 847 A1

# Pyrazolderivate,
# diese enthaltende pharmazeutische Zubereitungen und Verfahren zu ihrer Herstellung.

Die Erfindung betrifft neue Pyrazolderivate der allgemeinen Formel I

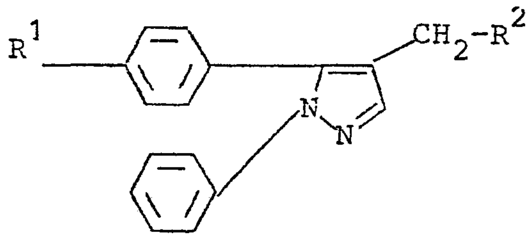

I

worin

$R^1$ H oder Cl,

$R^2$ 1-Methyl-4-piperidyloxycarbonyl, 2-(4-Phenylpiperazino)-äthoxycarbonyl, Benzoxazol-2-yl, Benzothiazol-2-yl, Tetrazol-5-yl oder 3-$R^3$-4-$R^4$-thiazolidin-2-yl,

$R^3$ H oder Acyl mit 1 - 7 C-Atomen und

$R^4$ H oder COOH

bedeuten

sowie ihre physiologisch unbedenklichen Salze.

Der Erfindung lag die Aufgabe zugrunde, neue Verbindungen mit wertvollen Eigenschaften aufzufinden, insbesondere solche, die zur Herstellung von Arzneimitteln verwendet werden können. Diese Aufgabe wurde durch die Bereitstellung der Verbindungen der Formel I gelöst.

Es wurde gefunden, daß die Verbindungen der Formel I bei guter Verträglichkeit wertvolle pharmakologische Eigenschaften besitzen. Insbesondere treten analgetische Wirkungen auf, die sich z.B. im Schmerztest (Writhing-Test) nach der Methode von Siegmund et al. (Proc. Soc. exp. Biol. (N.Y.) 95. (1957), Seiten 729 - 731) an Mäusen nachweisen lassen. Ferner zeigen sich beispielsweise cholesterinspiegelsenkende und triglyceridspiegelsenkende Wirkungen, nachweisbar im Serum von Ratten nach der Methode von Levine et al. (Automation in Analytical Chemistry, Technicon Symposium 1967, Mediad, New York, Seiten 25 - 28) bzw. nach der Methode von Noble und Campbell (Clin. Chem. 16 (1970), Seiten 166 - 170). Weiterhin können antiphlogistische Wirkungen (nachweisbar z.B. an Ratten nach der Methode von Winter et al., Proc. Soc. exp. Biol. (N.Y.) 111. (1962), Seite 544), ferner enzyminduzierende, fibrinolytische und thrombozyten-aggregationshemmende Wirkungen nach hierfür geläufigen Methoden beobachtet werden.

Die Verbindungen der Formel I können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

Gegenstand der Erfindung sind die Verbindungen der Formel I sowie ihre physiologisch unbedenklichen Salze.

Der Rest $R^1$ bedeutet vorzugsweise Cl. Der Rest $R^2$ steht vorzugsweise für 1-Methyl-4-piperidyloxycarbonyl oder 2-(4-Phenyl-piperazino)-äthoxycarbonyl. Die Reste $R^3$ und $R^4$ sind jeweils vorzugsweise H. Acyl (im Rest $R^3$)

ist bevorzugt Alkanoyl mit 1 - 7 C-Atomen oder Benzoyl, insbesondere Acetyl, ferner z.B. Formyl, Propionyl, Butyryl, Isobutyryl, Valeryl, Hexanoyl oder Heptanoyl.

Dementsprechend sind Gegenstand der Erfindung insbesondere diejenigen Verbindungen der Formel I, in denen mindestens einer der genannten Reste eine der vorstehend angegebenen bevorzugten Bedeutungen hat.

Eine besonders bevorzugte Gruppe von Verbindungen sind diejenigen Verbindungen der Formel I, in denen $R^1$ Cl und $R^2$ 1-Methyl-4-piperidyloxycarbonyl oder 2-(4-Phenylpiperazino)-äthoxycarbonyl bedeutet, sowie deren physiologisch unbedenkliche Salze.

Diejenigen Verbindungen der Formel I, in welchen der Rest $R^2$ 1-Methyl-4-piperidyloxycarbonyl oder 3-$R^3$-4-$R^4$-thiazolidin-2-yl bedeutet, besitzen ein bzw. zwei asymmetrische Kohlenstoffatome. Daher können sie als Racemate, falls mehrere asymmetrische Kohlenstoffatome vorhanden sind, auch als Gemische mehrerer Racemate sowie in verschiedenen optisch aktiven Formen vorliegen. Die Formel I umschließt alle diese racemischen und optisch aktiven Formen sowie ihre Gemische.

Gegenstand der Erfindung ist ferner ein Verfahren zur Herstellung der Pyrazolderivate der Formel I sowie ihrer physiologisch unbedenklichen Salze, dadurch gekennzeichnet, daß man eine Verbindung der allgemeinen Formel II

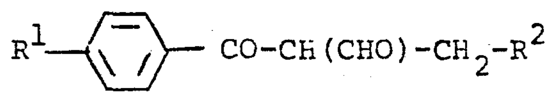 II

worin

$R^1$ und $R^2$ die angegebene Bedeutung haben

oder eines ihrer reaktionsfähigen Derivate mit Phenylhydrazin oder einem seiner Salze umsetzt, oder daß man zur Herstellung einer Verbindung der Formel I, worin $R^2$ eine 1-Methyl-4-piperidyloxycarbonyl-, 2-(4-Phenylpiperazino)-äthoxycarbonyl-, Benzoxazol-2-yl- oder Benzothiazol-2-ylgruppe bedeutet,

eine Carbonsäure der allgemeinen Formel III

R-COOH III

worin

R die Gruppe 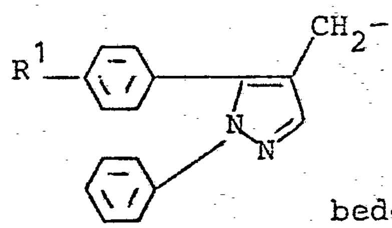 bedeutet und

$R^1$ die angegebene Bedeutung hat

oder eines ihrer reaktionsfähigen Derivate mit einer Verbindung der allgemeinen Formel IV

$H-R^5$ IV

worin

$R^5$ 1-Methyl-4-piperidyloxy, 2-(4-Phenylpiperazino)-äthoxy, 2-Aminophenoxy oder 2-Aminophenylthio bedeutet

oder einem ihrer reaktionsfähigen Derivate umsetzt,

oder daß man zur Herstellung einer Verbindung der allgemeinen Formel I, worin $R^2$ eine Tetrazol-5-ylgruppe bedeutet,

ein Nitril der allgemeinen Formel V

R-CN V

worin

R die angegebene Bedeutung hat

oder eines seiner reaktionsfähigen Derivate mit Stickstoffwasserstoffsäure oder einem ihrer Salze

oder ein Amidrazon der allgemeinen Formel VI

$$R{-}C({=}NH){-}NHNH_2 \qquad VI$$

worin

R die angegebene Bedeutung hat

mit salpetriger Säure oder einem ihrer Salze umsetzt,

oder daß man zur Herstellung einer Verbindung der allgemeinen Formel I, worin $R^2$ eine Benzothiazol-2-yl- oder eine 3-$R^3$-4-$R^4$-thiazolidin-2-yl-gruppe bedeutet, einen Aldehyd der allgemeinen Formel VII

R-CHO VII

worin

R die angegebene Bedeutung hat,

oder eines seiner reaktionsfähigen Derivate mit einer Verbindung der allgemeinen Formel VIII

$$R^6{-}SH \qquad VIII$$

worin

$R^6$ o-$H_2N$-$C_6H_4$- oder $NHR^3$-$CHR^4$-$CH_2$- bedeutet und

$R^3$ und $R^4$ die angegebene Bedeutung haben

oder einem ihrer reaktionsfähigen Derivate umsetzt, wobei man, falls $R^6$ eine o-$H_2N$-$C_6H_4$-Gruppe ist, in Gegenwart eines Oxydationsmittels arbeitet,

oder daß man zur Herstellung einer Verbindung der allgemeinen Formel I, worin $R^2$ eine 3-$R^3$-4-$R^4$-thiazolidin-2-yl-Gruppe und $R^3$ eine Acylgruppe mit 1 - 7 C-Atomen bedeutet, eine Verbindung der allgemeinen Formel I, worin $R^2$ eine 3-$R^3$-4-$R^4$-thiazolidin-2-yl-Gruppe und $R^3$ ein H-Atom bedeutet, mit einem Acylierungsmittel behandelt und/oder daß man gegebenenfalls eine Base bzw. Säure der Formel I durch Behandeln mit einer Säure bzw. Base in eines ihrer physiologisch unbedenklichen Salze überführt.

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Die Ausgangsstoffe der Formel II sind neu. Sie sind z.B. erhältlich durch Formylierung von Ketonen der allgemeinen Formel $p-R^1-C_6H_4-CO-CH_2-CH_2-R^2$, z.B. mit Äthylformiat; diese sind ihrerseits auf verschiedenen Wegen zugänglich, z.B. in Analogie zu den weiter unten beschriebenen Methoden, z.B. (falls $R^2 = R^5$) durch Umsetzung von 3-Benzoyl- oder 3-p-Chlorbenzoyl-propionsäure oder einem ihrer reaktionsfähigen Derivate mit einer Verbindung der allgemeinen Formel $H-R^5$ (IV) oder einem ihrer reaktionsfähigen Derivate, oder (falls $R^2$ = Tetrazol-5-yl) durch Umsetzung eines Nitrils der genannten Ketosäuren oder eines seiner reaktionsfähigen Derivate mit Stickstoffwasserstoffsäure oder einem ihrer Salze oder (falls $R^2$ = Benzothiazol-2-yl oder 3-$R^3$-4-$R^4$-thiazolidin-2-yl) durch Reaktion eines entsprechenden Ketoaldehyds oder eines seiner reaktionsfähigen Derivate mit einer Verbindung der allgemeinen Formel $R^6-SH$ (VIII).

Als reaktionsfähige Derivate der Verbindungen der Formel II eignen sich vorzugsweise die entsprechenden Enoläther, Enolester oder Enamine, die in üblicher Weise aus den Verbindungen II erhältlich sind. Besonders bevorzugt sind die Dimethylamino-enamine der allgemeinen Formel $p-R^1-C_6H_4-CO-C(CH_2-R^2)=CH-N(CH_3)_2$; diese sind vorzugsweise erhältlich durch Umsetzung der oben genannten Ketone der Formel $p-R^1-C_6H_4-CO-CH_2-CH_2-R^2$ mit Dimethylformamid-dimethylacetal in Gegenwart von Essigsäure.

Die Umsetzung der Verbindungen der Formel II oder ihrer reaktionsfähigen Derivate mit Phenylhydrazin bzw. dessen Salzen kann lösungsmittelfrei oder in einem inerten Lösungsmittel bei Temperaturen zwischen etwa 0 und etwa 160°, vorzugsweise zwischen 70 und 120° durchgeführt

werden. Als Lösungsmittel eignen sich z. B. Alkohole wie Methanol, Äthanol, n-Butanol, Kohlenwasserstoffe wie Benzol, Toluol oder Xylol, Chlorkohlenwasserstoffe wie Chlorbenzol, Äther wie Diäthyläther, Tetrahydrofuran (THF) oder Dioxan, Äthylenglykol- oder Diäthylenglykol-mono- oder -dialkyläther wie Methylglykol oder Äthylglykol, aliphatische Carbonsäuren wie Essigsäure, Amide wie Dimethylformamid (DMF) oder N-Methylpyrrolidon, Sulfoxide wie Dimethylsulfoxid. Die Reaktionszeiten liegen in der Regel zwischen 30 Minuten und 8 Stunden. Die Umsetzung wird vorzugsweise in etwa neutralem Medium vorgenommen, z.B. unter Zusatz von Essigsäure oder, falls ein Salz des Phenylhydrazins verwendet wird, unter Zusatz von Natriumacetat.

Verbindungen der Formel I, in denen $R^2 = R^5$ ist, können zweckmäßig durch Umsetzung von Carbonsäuren der Formel III oder ihren reaktionsfähigen Derivaten mit Verbindungen der Formel IV oder ihren reaktionsfähigen Derivaten hergestellt werden. Als reaktionsfähige Derivate von III eignen sich z.B. die entsprechenden Säurehalogenide, insbesondere die Chloride, ferner die Ester, insbesondere Alkylester, in denen die Alkylgruppe 1 - 4 C-Atome enthält, ferner Nitrile, Amide, Anhydride oder Imidoester. Als funktionelle Derivate der Alkohole oder Phenole der Formel IV sind insbesondere die entsprechenden Metall-alkoholate bzw. -phenolate geeignet, insbesondere die Alkalimetall-alkoholate oder -phenolate. Die Ausgangsstoffe III und IV sind bekannt, ihre reaktionsfähigen Derivate können leicht nach an sich bekannten Methoden daraus erhalten werden.

Eine Veresterung mit Carbonsäuren III mit 4-Hydroxy-1-methylpiperidin oder 2-(4-Phenylpiperazino)-äthanol kann in üblicher Weise in Gegenwart oder Abwesenheit eines zusätzlichen Lösungsmittels und/oder eines wasserabspaltenden Mittels erfolgen. Als Lösungsmittel eignen sich z.B. Kohlenwasserstoffe wie Benzol, Toluol oder Xylol, halogenierte Kohlenwasserstoffe wie Dichlormethan, Chloroform oder 1,2-Dichloräthan, Äther wie Tetrahydrofuran. Als wasserabspaltende Mittel haben sich insbesondere Carbodiimide wie Dicyclohexylcarbodiimid bewährt. Die Veresterung wird zweckmäßig bei Temperaturen zwischen etwa 0 und etwa 140° durchgeführt; sie ist in der Regel nach 30 Minuten bis 8 Stunden beendet.

Eine Umsetzung der Carbonsäuren III oder ihrer reaktionsfähigen Derivate, insbesondere ihrer Chloride, mit o-Aminophenol oder o-Amino-thiophenol erfolgt nach den üblichen Methoden der Benzoxazol- oder Benzthiazol-Synthesen. Verwendet man die freien Säuren III als Ausgangsstoffe, so arbeitet man zweckmäßig in Gegenwart eines wasserabspaltenden Mittels wie $P_2O_5$ oder Polyphosphorsäure; verwendet man die Chloride, so setzt man vorteilhaft eine Base zur Bindung des entstehenden Chlorwasserstoffs hinzu, z.B. Dimethylanilin oder Pyridin, welche gleichzeitig als Lösungsmittel dienen können. Die Reaktionstemperaturen liegen bei diesen Umsetzungen zweckmäßig zwischen etwa 20 und etwa 200°, vorzugsweise zwischen 100 und 180°. Die Reaktionszeiten liegen in der Regel zwischen etwa 30 Minuten und 8 Stunden.

0014847

Tetrazole der Formel I ($R^2$ = Tetrazol-5-yl) sind zweckmäßig erhältlich durch Umsetzung eines Nitrils der Formel V oder eines seiner reaktionsfähigen Derivate, z.B. eines entsprechenden Imidoesters, mit Stickstoffwasserstoffsäure oder einem ihrer Salze, zweckmäßig einem ihrer Alkalimetallsalze wie Natriumazid. Die Nitrile der Formel V und die entsprechenden Imidoester sind entweder bekannt, oder sie können mit Hilfe bekannter Methoden aus bekannten Verbindungen hergestellt werden. Die Umsetzung gelingt zweckmäßig in Gegenwart eines der oben genannten Lösungsmittel. Dimethylformamid ist als Lösungsmittel bevorzugt. Die Reaktionstemperaturen liegen zweckmäßig zwischen etwa 20 und etwa 200, vorzugsweise zwischen 80 und 150°. Die Imidoester werden zweckmäßig mit freier Stickstoffwasserstoffsäure in Benzol bei Siedetemperatur umgesetzt. Die Reaktionszeiten bei diesen Umsetzungen liegen in der Regel zwischen 1 Stunde und 10 Tagen.

Verbindungen der Formel I, worin $R^2$ eine Tetrazol-5-yl-Gruppe bedeutet, sind weiterhin in guten Ausbeuten erhältlich durch Umsetzung von Amidrazonen der Formel VI, die ihrerseits leicht aus den entsprechenden Nitrilen und Natriumhydrazid erhältlich sind, mit salpetriger Säure oder einem ihrer Salze, vorzugsweise einem ihrer Alkalimetallsalze wie Natriumnitrit. Diese Umsetzung gelingt beispielsweise in wässerig-alkoholischer, z.B. wässerig-äthanolischer Lösung bei Temperaturen zwischen etwa -10 und 20°, vorzugsweise zwischen 0 und 5°. Zweckmäßig tropft man eine wässerige Natriumnitritlösung zu einer angesäuerten alkoholischen Lösung des Amidrazons und läßt anschließend etwa 1 bis 48 Stunden bei 0 bis 5° stehen.

Umsetzung von Aldehyden der Formel VII mit Aminothiolen der Formel VIII führt zu Verbindungen der Formel I, worin $R^2$ eine Benzthiazol-2-yl- oder eine 3-$R^3$-4-$R^4$-thiazolidin-2-yl-Gruppe bedeutet, wobei man im ersten Fall in Gegenwart eines Oxydationsmittels arbeitet. Die Aldehyde der Formel VII sind leicht erhältlich durch Reduktion der entsprechenden Carbonsäuren der Formel III oder ihrer Ester oder durch Oxydation der entsprechenden Alkohole der Formel $R-CH_2OH$, die ihrerseits durch Reduktion der Säuren oder der Ester erhältlich sind. Die Aminothiole der Formel VIII sind entweder bekannt, oder sie können nach bekannten Methoden aus bekannten Ausgangsstoffen hergestellt werden. Anstelle der Ausgangsstoffe VII bzw. VIII können auch entsprechende reaktionsfähige Derivate eingesetzt werden, beispielsweise die Acetale, Hemithioacetale, Thioacetale, Acylale oder Thioacylale entsprechend VII, die Mercaptide, insbesondere die Alkalimetallmercaptide von VIII. Die Umsetzung von VII mit VIII wird zweckmäßig in Gegenwart eines inerten Lösungsmittels vorgenommen, z.B. eines der oben genanten Lösungsmittel, wobei die Umsetzung zu den Thiazolidinen besonders vorteilhaft in wässerig-alkoholischer, z.B. wässerig-äthanolischer schwach saurer Lösung bei Temperaturen zwischen etwa 0 und etwa 80, vorzugsweise zwischen 15 und 30° durchgeführt wird, die Umsetzung zu den Benzothiazolen in einem Kohlenwasserstoff wie Benzol oder Toluol in Gegenwart eines sauren Katalysators wie Salzsäure, Schwefelsäure oder p-Toluolsulfonsäure unter Luftzutritt bei Temperaturen zwischen etwa 50 und etwa 150, vorzugsweise zwischen 80 und 110°. Im letzten Fall ist die Gegenwart eines Oxydationsmittels erforderlich, wobei jedoch in der Regel der Zutritt von Luft oder das Einleiten von Sauerstoff genügt.

Verbindungen der Formel I, worin $R^2$ eine 3-Acyl-4-$R^4$-thiazolidin-2-yl-Gruppe bedeutet, sind weiterhin durch übliche N-Acylierung der entsprechenden NH-Verbindung (I, $R^2$ = 4-$R^4$-thiazolidin-2-yl) erhältlich. Als Acylierungsmittel eignen sich zweckmäßig die Halogenide (z.B. Chloride oder Bromide) oder Anhydride von Carbonsäuren mit 1 - 7 C-Atomen, z.B. Acetanhydrid, Propionylchlorid, Isobutyrylbromid, Ameisensäure-essigsäure-anhydrid oder Benzoylchlorid. Der Zusatz einer Base wie Pyridin oder Triäthylamin bei der Acylierung ist möglich, aber nicht notwendig. Die Acylierung wird zweckmäßig in Gegenwart oder Abwesenheit eines der genannten inerten Lösungsmittel, z.B. Benzol, bei Temperaturen zwischen etwa 0 und etwa 160°, vorzugsweise zwischen etwa 20 und etwa 120° vorgenommen. Als Lösungsmittel kann auch die genannte Base, z.B. Pyridin, oder ein Überschuß des Acylierungsmittels dienen.

Eine Base der Formel I kann mit einer Säure in das zugehörige Säureadditionssalz übergeführt werden. Für diese Umsetzung kommen solche Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, ferner organische Säuren, insbesondere aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diäthylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure,

Citronensäure, Gluconsäure, Ascorbinsäure, Nicotinsäure, Isonicotinsäure, Methan- oder Äthansulfonsäure, Äthandisulfonsäure, 2-Hydroxyäthansulfonsäure, Benzolsulfonsäure, p-Toluolsulfonsäure, Naphthalin-mono- und -disulfonsäuren, Laurylschwefelsäure.

Die Säuren der Formel I ($R^4$ = COOH) können durch Umsetzung mit einer Base in eines ihrer physiologisch unbedenklichen Metall- bzw. Ammoniumsalze übergeführt werden. Als Salze kommen insbesondere die Natrium-, Kalium-, Magnesium-, Calcium- und Ammoniumsalze in Betracht, ferner substituierte Ammoniumsalze, z.B. die Dimethyl-, Diäthyl- und Diisopropylammonium-, Monoäthanol-, Diäthanol- und Triäthanolammonium-, Cyclohexylammonium-, Dicyclohexylammonium- und Dibenzyläthylendiammonium-Salze.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyäthylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder. Sofern die Arzneimittel in Form abgeteilter Pulver verabreicht werden sollen, so sind auch die Verpackungsmaterialien wie Papierbriefchen oder Papierkapseln geeignete Trägerstoffe. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Gegenstand der Erfindung ist ferner die Verwendung der Verbindungen der Formel I bei der Bekämpfung von Krankheiten, insbesondere von schmerzhaften Erkrankungen des Bewegungsapparates (Rheuma) und bei Schmerzen an anderen Organen, sowie ihre Verwendung bei der therapeutischen Behandlung des menschlichen oder tierischen Körpers.

Dabei werden die erfindungsgemäßen Substanzen in der Regel in Analogie zu bekannten, im Handel befindlichen Analgetika oder Antiphlogistika (z.B. Metamizol, Acetylsalicylsäure, Paracetamol), verabreicht, vorzugsweise in Dosierungen zwischen etwa 1 und 500 mg, insbesondere zwischen 5 und 50 mg pro Dosierungseinheit. Die tägliche Dosierung liegt vorzugsweise zwischen etwa 0,02 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Jede der in den folgenden Beispielen genannten Verbindungen der Formel I ist zur Herstellung von pharmazeutischen Zubereitungen besonders geeignet.

In den nachfolgenden Beispielen bedeutet "übliche Aufarbeitung":

Man gibt, falls erforderlich, Wasser hinzu, extrahiert mit einem organischen Lösungsmittel wie Benzol, Chloroform oder Dichlormethan, trennt ab, trocknet die organische Phase über Natriumsulfat, filtriert, dampft ein und reinigt durch Chromatographie und/oder Kristallisation.

Beispiel 1

Ein Gemisch aus 33,8 g 3-p-Chlorbenzoyl-3-formyl-propionsäure-(1-methyl-4-piperidyl)-ester /erhältlich durch Veresterung von 3-p-Chlorbenzoylpropionsäure mit 4-Hydroxy-1-methylpiperidin zu 3-p-Chlorbenzoylpropionsäure-(1-methyl-4-piperidyl)-ester und anschließende Kondensation mit Äthylformiat7, 11,9 g Phenylhydrazin, 7 ml Essigsäure und 350 ml Äthanol wird 2 Stunden gekocht und anschließend eingedampft. Nach üblicher Aufarbeitung erhält man (5-p-Chlorphenyl-1-phenyl-pyrazol-4-yl)-essigsäure-(1-methyl-4-piperidyl)-ester, F. 90 - 93°. Hydrochlorid, F. 234 - 236°.

Beispiele 2 bis 16

Analog Beispiel 1 erhält man aus

3-p-Chlorbenzoyl-3-formyl-propionsäure-/2-(4-phenylpiperazino)-äthyl7-ester
2-(2-p-Chlorbenzoyl-3-oxopropyl)-benzoxazol
2-(2-p-Chlorbenzoyl-3-oxopropyl)-benzothiazol
5-(2-p-Chlorbenzoyl-3-oxopropyl)-tetrazol
2-(2-p-Chlorbenzoyl-3-oxopropyl)-thiazolidin
2-(2-p-Chlorbenzoyl-3-oxopropyl)-thiazolidin-4-carbonsäure
2-(2-p-Chlorbenzoyl-3-oxopropyl)-3-acetyl-thiazolidin
3-Benzoyl-3-formyl-propionsäure-(1-methyl-4-piperidyl)-ester bzw.
3-Benzoyl-3-formyl-propionsäure-/2-(4-phenylpiperazino)-äthyl7-ester
2-(2-Benzoyl-3-oxopropyl)-benzoxazol
2-(2-Benzoyl-3-oxopropyl)-benzothiazol
5-(2-Benzoyl-3-oxopropyl)-tetrazol
2-(2-Benzoyl-3-oxopropyl)-thiazolidin

2-(2-Benzoyl-3-oxopropyl)-thiazolidin-4-carbonsäure

2-(2-Benzoyl-3-oxopropyl)-3-acetyl-thiazolidin

mit Phenylhydrazin:

2. (5-p-Chlorphenyl-1-phenyl-pyrazol-4-yl)-essigsäure-/2-(4-phenylpiperazino)-äthyl/-ester, F. 78-80°. Hemisuccinat (saures Succinat), F. 105-107°.

3. 5-p-Chlorphenyl-1-phenyl-4-(benzoxazol-2-ylmethyl)-pyrazol, F. 137-139°.

4. 5-p-Chlorphenyl-1-phenyl-4-(benzothiazol-2-ylmethyl)-pyrazol, F. 135-137°.

5. 5-p-Chlorphenyl-1-phenyl-4-(tetrazol-5-ylmethyl)-pyrazol, F. 230-232°.

6. 5-p-Chlorphenyl-1-phenyl-4-(thiazolidin-2-ylmethyl)-pyrazol, F. 147-148°. Dihydrochlorid, F. 173-176°. Phosphat, F. 170-172°.

7. 5-p-Chlorphenyl-1-phenyl-4-(4-carboxythiazolidin-2-ylmethyl)-pyrazol, F. 175-177°.

8. 4-(3-Acetylthiazolidin-2-ylmethyl)-5-p-chlorphenyl-1-phenyl-pyrazol, F. 121-122°.

9. (1,5-Diphenyl-pyrazol-4-yl)-essigsäure-(1-methyl-4-piperidyl)-ester.

10. (1,5-Diphenyl-pyrazol-4-yl)-essigsäure-/2-(4-phenylpiperazino)-äthyl/-ester.

11. 1,5-Diphenyl-4-(benzoxazol-2-ylmethyl)-pyrazol.

12. 1,5-Diphenyl-4-(benzothiazol-2-ylmethyl)-pyrazol.

13. 1,5-Diphenyl-4-(tetrazol-5-ylmethyl)-pyrazol.

14. 1,5-Diphenyl-4-(thiazolidin-2-ylmethyl)-pyrazol.

15. 1,5-Diphenyl-4-(4-carboxythiazolidin-2-ylmethyl)-pyrazol.

16. 4-(3-Acetylthiazolidin-2-ylmethyl)-1,5-diphenylpyrazol.

Beispiel 17

Man erwärmt 35,1 g 3-p-Chlorbenzoyl-4-dimethylamino-3-butensäure-(1-methyl-4-piperidyl)-ester /erhältlich durch Veresterung von 3-p-Chlorbenzoyl-propionsäure mit 1-Methylpiperidin-4-ol zu 3-p-Chlorbenzoyl-propionsäure-(1-methyl-4-piperidyl)-ester und anschließende Reaktion mit Dimethylformamid-dimethylacetal in Gegenwart von Essigsäure bei 120°7 und 12 g Phenylhydrazin 4 Stunden auf 100°, arbeitet wie üblich auf (Benzol/Wasser) und erhält 5-p-Chlorphenyl-1-phenyl-pyrazol-4-yl-essigsäure-(1-methyl-4-piperidyl)-ester, F. 90-93°. Hydrochlorid, F. 234-235°.

Beispiele 18 bis 20

Analog Beispiel 17 erhält man aus

3-p-Chlorbenzoyl-4-dimethylamino-3-butensäure-/2-(4-phenylpiperazino)-äthyl7-ester
3—Benzoyl-4-dimethylamino-3-butensäure-(1-methyl-4-piperidyl)-ester
3-Benzoyl-4-dimethylamino-3-butensäure-/2-(4-phenylpiperazino)-äthyl7-ester

mit Phenylhydrazin:

18. (5-p-Chlorphenyl-1-phenyl-pyrazol-4-yl)-essigsäure-/2-(4-phenylpiperazino)-äthyl7-ester, F. 78-80°. Hemisuccinat, F. 105-107°.

19. (1,5-Diphenyl-pyrazol-4-yl)-essigsäure-(1-methyl-4-piperidyl)-ester.

20. (1,5-Diphenyl-pyrazol-4-yl)-essigsäure-/2-(4-phenylpiperazino)-äthyl7-ester.

Beispiel 21

Zu einer Lösung von 3,13 g (5-p-Chlorphenyl-1-phenyl-pyrazol-4-yl)-essigsäure in 25 ml trockenem THF gibt man bei 5 - 10° eine Lösung von 2,27 g Dicyclohexylcarbodiimid in 10 ml trockenem THF langsam hinzu. Man rührt 15 Minuten bei 5 - 10°, gibt anschließend eine Lösung von 1,23 g 4-Hydroxy-1-methylpiperidin in 10 ml trockenem THF bei 5 - 10° hinzu, rührt 3 Stunden bei 20°, arbeitet wie üblich auf und erhält (5-p-Chlorphenyl-1-phenyl-pyrazol-4-yl)-essigsäure-(1-methyl-4-piperidyl)-ester, F. 90-93°. Hydrochlorid, F.234-236°.

Beispiele 22 bis 24

Analog Beispiel 21 erhält man aus (5-p-Chlorphenyl-1-phenylpyrazol-4-yl)-essigsäure oder aus (1,5-Diphenyl-pyrazol-4-ylessigsäure) mit 2-(4-Phenylpiperazino)-äthanol oder 4-Hydroxy-1-methylpiperidin:

22. (5-p-Chlorphenyl-1-phenyl-pyrazol-4-yl)-essigsäure-/2-(4-phenylpiperazino)-äthyl7-ester, F. 78-80°. Hemisuccinat, F. 106-107°.

23. (1,5-Diphenyl-pyrazol-4-yl)-essigsäure-(1-methyl-4-piperidyl)-ester.

24. (1,5-Diphenyl-pyrazol-4-yl)-essigsäure-/2-(4-phenylpiperazino)-äthyl7-ester.

Beispiel 25

Ein Gemisch aus 10,9 g o-Aminophenol, 33,1 g (5-p-Chlorphenyl-1-phenyl-pyrazol-4-yl)-acetylchlorid und 100 ml Dimethylanilin wird 1 Stunde auf 100°, dann 1 Stunde auf 160° erhitzt. Man kühlt ab, arbeitet wie üblich auf und erhält 5-p-Chlorphenyl-1-phenyl-4-(benzoxazol-2-ylmethyl)-pyrazol, F. 137-139°.

Beispiel 26

Analog Beispiel 25 erhält man mit (1,5-Diphenyl-pyrazol-4-yl)-acetylchlorid das 1,5-Diphenyl-4-(benzoxazol-2-ylmethyl)-pyrazol.

Beispiel 27

Ein Gemisch aus 31,3 g (5-p-Chlorphenyl-1-phenyl-pyrazol-4-yl)-essigsäure, 10,9 g o-Aminophenol, und 400 ml Polyphosphorsäure wird 2 Stunden auf 175° erhitzt. Nach dem Abkühlen und üblicher Aufarbeitung erhält man 5-p-Chlorphenyl-1-phenyl-4-(benzoxazol-2-ylmethyl)-pyrazol, F. 137-139°.

Beispiel 28

Analog Beispiel 27 erhält man mit (1,5-Diphenyl-pyrazol-4-yl)-essigsäure das 1,5-Diphenyl-4-(benzoxazol-2-ylmethyl)-pyrazol.

Beispiel 29

Ein Gemisch aus 12,5 g 2-Amino-thiophenol, 33,1 g (5-p-Chlorphenyl-1-phenyl-pyrazol-4-yl)-acetylchlorid und 100 ml Dimethylanilin wird 1 Stunde auf 100°, dann 1 Stunde auf 140° erhitzt. Man kühlt ab, arbeitet wie üblich auf und erhält 5-p-Chlorphenyl-1-phenyl-4-(benzothiazol-2-ylmethyl)-pyrazol, F. 135-137°.

Beispiel 30

Analog Beispiel 29 erhält man mit (1,5-Diphenyl-pyrazol-4-yl)-acetylchlorid das 1,5-Diphenyl-4-(benzothiazol-2-ylmethyl)-pyrazol.

Beispiel 31

Ein Gemisch aus 29,4 g (5-p-Chlorphenyl-1-phenyl-pyrazol-4-yl)-acetonitril, 8,06 g Natriumazid, 6,64 g $NH_4Cl$ und 140 ml trockenem DMF wird unter Rühren 17 Stunden auf 120° erhitzt und anschließend eingedampft. Nach üblicher Aufarbeitung erhält man 5-p-Chlorphenyl-1-phenyl-4-(tetrazol-5-ylmethyl)-pyrazol, F. 230-232°.

Beispiel 32

Analog Beispiel 31 erhält man mit (1,5-Diphenylpyrazol-4-yl)-acetonitril das 1,5-Diphenyl-4-(tetrazol-5-ylmethyl)-pyrazol.

Beispiel 33

Man kocht 34 g (5-p-Chlorphenyl-1-phenylpyrazol-4-yl)-essigsäure-imidoäthylester mit 120 ml einer Lösung von 0,15 Mol Stickstoffwasserstoffsäure in Benzol 2 Stunden, gibt dann weitere 40 ml der Stickstoffwasserstoffsäure-Lösung hinzu (insgesamt also 0,2 Mol), kocht weitere 2 Stunden, kühlt ab, arbeitet wie üblich auf und erhält 5-p-Chlorphenyl-1-phenyl-4-tetrazol-5-ylmethyl)-pyrazol, F. 230-232°.

Beispiel 34

Eine Lösung von 0,75 g $NaNO_2$ in 10 ml Wasser wird zugetropft zu einer Lösung von 3,26 g 5-p-Chlorphenyl-1-phenyl-pyrazol-4-yl-essigsäure-amidrazon (erhältlich aus dem Nitril und Natriumhydrazid) in 250 ml Aethanol und 3 g 38%iger Salzsäure. Man läßt 24 Stunden bei 0° stehen, neutralisiert mit Natronlauge, dampft ein, arbeitet wie üblich auf und erhält 5-p-Chlorphenyl-1-phenyl-4-(tetrazol-5-yl-methyl)-pyrazol, F. 230-232°.

Beispiel 35

Ein Gemisch aus 29,7 g (5-p-Chlorphenyl-1-phenyl-pyrazol-4-yl)-acetaldehyd /F. 130 - 132°; erhältlich durch Reduktion von (5-p-Chlorphenyl-1-phenyl-pyrazol-4-yl)-essigsäureäthylester mit $LiAlH_4$ zu 5-p-Chlorphenyl-4-(2-hydroxyäthyl)-1-phenylpyrazol (F. 120 - 122°) und Oxydation mit Dimethylsulfoxid/Dicyclohexylcarbodiimid in Benzol bei 25°7, 13,5 g Cysteaminhydrochlorid, 8,67 g $CH_3COOK$, 75 ml Wasser und 150 ml Aethanol wird 2 Stunden bei 25° gerührt. Man arbeitet wie üblich auf (Wasser/Äthylacetat) und erhält 5-p-Chlorphenyl-1-phenyl-4-(thiazolidin-2-yl-methyl)-pyrazol, F. 147-148°, Dihydrochlorid, F. 173-176°. Phosphat, F. 170-172°.

Beispiel 36

Analog Beispiel 35 erhält man mit (1,5-Diphenyl-pyrazol-4-yl)-acetaldehyd das 1,5-Diphenyl-4-(thiazolidin-2-yl-methyl)-pyrazol.

Beispiele 37 und 38

Analog Beispiel 35 und 36 erhält man mit Cysteinhydrochlorid:

37. 5-p-Chlorphenyl-1-phenyl-4-(4-carboxythiazolidin-2-ylmethyl)-pyrazol, F. 175-177°.

38. 1,5-Diphenyl-4-(4-carboxythiazolidin-2-ylmethyl)-pyrazol.

Beispiel 39

Ein Gemisch von 29,7 g (5-p-Chlorphenyl-1-phenyl-pyrazol-4-yl)-acetaldehyd, 12,5 g 2-Amino-thiophenol, 18 g p-Toluolsulfonsäure und 500 ml Toluol wird 3 Stunden mit Wasserabscheider unter Luftzutritt gekocht.
Übliche Aufarbeitung liefert 5-p-Chlorphenyl-1-phenyl-4-(benzothiazol-2-ylmethyl)-pyrazol, F. 135-137°.

Beispiel 40

Ein Gemisch aus 3,56 g 5-p-Chlorphenyl-1-phenyl-4-(thiazolidin-2-yl-methyl)-pyrazol und 55 ml Acetanhydrid wird 3 Stunden gekocht. Man gibt danach 55 ml Pyridin hinzu, erhitzt weitere 5 Stunden auf 50°, arbeitet wie üblich auf und erhält 4-(3-Acetylthiazolidin-2-ylmethyl)-5-p-chlorphenyl-1-phenylpyrazol, F. 121-122°.

Beispiele 41 bis 47

Analog Beispiel 40 erhält man mit den entsprechenden Säureanhydriden

41. 4-(3-Propionyl-thiazolidin-2-ylmethyl)-5-p-chlorphenyl-1-phenylpyrazol.
42. 4-(3-Butyryl-thiazolidin-2-ylmethyl)-5-p-chlorphenyl-1-phenylpyrazol.
43. 4-(3-Isobutyryl-thiazolidin-2-ylmethyl)-5-p-chlorphenyl-1-phenylpyrazol.
44. 4-(3-Valeryl-thiazolidin-2-ylmethyl)-5-p-chlorphenyl-1-phenylpyrazol.

45. 4-(3-Hexanoyl-thiazolidin-2-ylmethyl)-5-p-chlorphenyl-1-phenylpyrazol.

46. 4-(3-Heptanoyl-thiazolidin-2-ylmethyl)-5-p-chlorphenyl-1-phenylpyrazol.

47. 4-(3-Benzoyl-thiazolidin-2-ylmethyl)-5-p-chlorphenyl-1-phenylpyrazol.

Beispiele 48 bis 55

Analog Beispiel 40 bis 47 erhält man aus 1,5-Diphenyl-4-(thiazolidin-2-ylmethyl)-pyrazol:

48. 4-(3-Acetyl-thiazolidin-2-ylmethyl)-1,5-diphenylpyrazol.

49. 4-(3-Propionyl-thiazolidin-2-ylmethyl)-1,5-diphenylpyrazol.

50. 4-(3-Butyryl-thiazolidin-2-ylmethyl)-1,5-diphenylpyrazol.

51. 4-(3-Isobutyryl-thiazolidin-2-ylmethyl)-1,5-diphenylpyrazol.

52. 4-(3-Valeryl-thiazolidin-2-ylmethyl)-1,5-diphenylpyrazol.

53. 4-(3-Hexanoyl-thiazolidin-2-ylmethyl)-1,5-diphenylpyrazol.

54. 4-(3-Heptanoyl-thiazolidin-2-ylmethyl)-1,5-diphenylpyrazol.

55. 4-(3-Benzoyl-thiazolidin-2-ylmethyl)-1,5-diphenylpyrazol.

Die nachstehenden Beispiele betreffen pharmazeutische Zubereitungen, die Pyrazolderivate der Formel I oder ihre Salze enthalten.

Beispiel A: Tabletten

Ein Gemisch von 1 kg 5-p-Chlorphenyl-1-phenyl-4-(thiazolidin-2-yl-methyl)-pyrazol-dihydrochlorid, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 50 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

10 kg 5-p-Chlorphenyl-1-phenyl-pyrazol-4-yl-essigsäure-(1-methyl-4-piperidyl)-ester-hydrochlorid werden in üblicher Weise in Hartgelatinekapseln gefüllt, so daß jede Kapsel 50 mg Wirkstoff enthält.

Beispiel D: Ampullen

Eine Lösung von 1 kg 5-p-Chlorphenyl-1-phenyl-pyrazol-4-yl-essigsäure-[2-(4-phenylpiperazino)-äthyl]-ester-hemisuccinat in 30 l zweifach destilliertem Wasser wird steril filtriert, in Ampullen abgefüllt, unter sterilen Bedingungen lyophilisiert und steril verschlossen. Jede Ampulle enthält 20 mg Wirkstoff.

Merck Patent Gesellschaft
mit beschränkter Haftung
Darmstadt

Patentansprüche:

1. Pyrazolderivate der allgemeinen Formel I

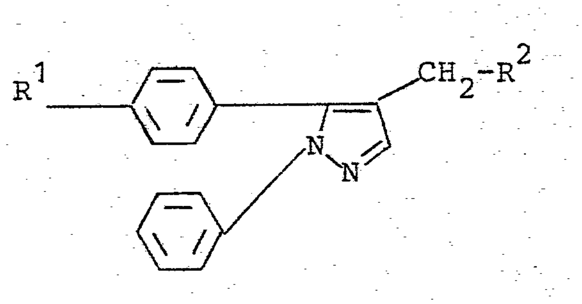

I

worin

$R^1$ H oder Cl,

$R^2$ 1-Methyl-4-piperidyloxycarbonyl, 2-(4-Phenylpiperazino)-äthoxycarbonyl, Benzoxazol-2-yl, Benzothiazol-2-yl, Tetrazol-5-yl oder 3-$R^3$-4-$R^4$-thiazolidin-2-yl,

$R^3$ H oder Acyl mit 1 - 7 C-Atomen und

$R^4$ H oder COOH

bedeuten

sowie ihre physiologisch unbedenkliche Salze.

2. a) 5-p-Chlorphenyl-1-phenyl-pyrazol-4-yl-essigsäure-(1-methyl-4-piperidyl)-ester;

b) 5-p-Chlorphenyl-1-phenyl-pyrazol-4-yl-essigsäure-[2-(4-phenylpiperazino)-äthyl]-ester;

c) 5-p-Chlorphenyl-1-phenyl-4-(tetrazol-5-ylmethyl)-pyrazol;

d) 5-p-Chlorphenyl-1-phenyl-4-(thiazolidin-2-ylmethyl)-pyrazol.

3. Verfahren zur Herstellung von Pyrazolderivaten der allgemeinen Formel I

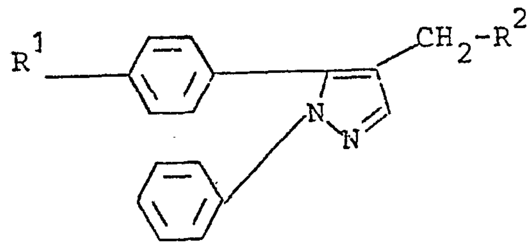

I

worin

$R^1$ H oder Cl,

$R^2$ 1-Methyl-4-piperidyloxycarbonyl, 2-(4-Phenylpiperazino)-äthoxycarbonyl, Benzoxazol-2-yl, Benzothiazol-2-yl, Tetrazol-5-yl oder $3\text{-}R^3\text{-}4\text{-}R^4$-thiazolidin-2-yl,

$R^3$ H oder Acyl mit 1 - 7 C-Atomen und

$R^4$ H oder COOH

bedeuten

sowie von ihren physiologisch unbedenklichen Salzen, dadurch gekennzeichnet, daß man eine Verbindung der allgemeinen Formel II

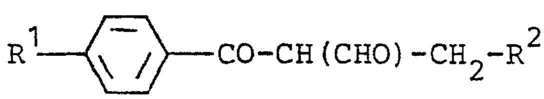

II

worin

$R^1$ und $R^2$ die angegebene Bedeutung haben,

oder eines ihrer reaktionsfähigen Derivate mit Phenylhydrazin oder einem seiner Salze umsetzt, oder daß man zur Herstellung einer Verbindung der Formel I, worin $R^2$ eine 1-Methyl-4-piperidyloxycarbonyl-, 2-(4-Phenylpiperazino)-äthoxycarbonyl-, Benzoxazol-2-yl- oder Benzothiazol-2-ylgruppe bedeutet,

eine Carbonsäure der allgemeinen Formel III

R-COOH                III

worin

R die Gruppe 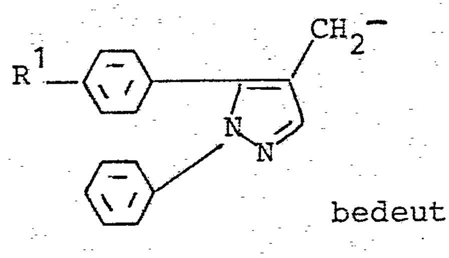 bedeutet und

$R^1$ die angegebene Bedeutung hat

oder eines ihrer reaktionsfähigen Derivate mit einer Verbindung der allgemeinen Formel IV

$H-R^5$                IV

worin

$R^5$ 1-Methyl-4-piperidyloxy, 2-(4-Phenyl-piperazino)-äthoxy, 2-Aminophenoxy oder 2-Aminophenylthio bedeutet

oder einem ihrer reaktionsfähigen Derivate umsetzt,

oder daß man zur Herstellung einer Verbindung der allgemeinen Formel I, worin $R^2$ eine Tetrazol-5-yl-gruppe bedeutet,

ein Nitril der allgemeinen Formel V

R-CN                V

worin

R die angegebene Bedeutung hat

oder eines seiner reaktionsfähigen Derivate mit Stickstoffwasserstoffsäure oder einem ihrer Salze

oder ein Amidrazon der allgemeinen Formel VI

$$R-C(=NH)-NHNH_2 \qquad VI$$

worin

R die angegebene Bedeutung hat

mit salpetriger Säure oder einem ihrer Salze umsetzt,

oder daß man zur Herstellung einer Verbindung der allgemeinen Formel I, worin $R^2$ eine Benzothiazol-2-yl- oder eine 3-$R^3$-4-$R^4$-thiazolidin-2-yl-gruppe bedeutet, einen Aldehyd der allgemeinen Formel VII

$$R-CHO \qquad VII$$

worin

R die angegebene Bedeutung hat,

oder eines seiner reaktionsfähigen Derivate mit einer Verbindung der allgemeinen Formel VIII

$$R^6-SH \qquad VIII$$

worin

$R^6$ o-$H_2N-C_6H_4$- oder $NHR^3-CHR^4-CH_2$- bedeutet und

$R^3$ und $R^4$ die angegebene Bedeutung haben

oder einem ihrer reaktionsfähigen Derivate umsetzt, wobei man, falls $R^6$ eine o-$H_2N-C_6H_4$-Gruppe ist, in Gegenwart eines Oxydationsmittels arbeitet,

oder daß man zur Herstellung einer Verbindung der allgemeinen Formel I, worin $R^2$ eine 3-$R^3$-4-$R^4$-thiazolidin-2-yl-Gruppe und $R^3$ eine Acylgruppe mit 1 - 7 C-Atomen bedeutet, eine Verbindung der allge-

meinen Formel I, worin $R^2$ eine 3-$R^3$-4-$R^4$-thiazolidin-2-yl-Gruppe und $R^3$ ein H-Atom bedeutet, mit einem Acylierungsmittel behandelt und/oder daß man gegebenenfalls eine Base bzw. Säure der Formel I durch Behandeln mit einer Säure bzw. Base in eines ihrer physiologisch unbedenklichen Salze überführt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

0014847

Europäisches Patentamt

# EUROPÄISCHER RECHERCHENBERICHT

Nummer der Anmeldung

EP 80100294.0

| EINSCHLÄGIGE DOKUMENTE | | | KLASSIFIKATION DER ANMELDUNG (Int.Cl.X 3) |
|---|---|---|---|
| Kategorie | Kennzeichnung des Dokuments mit Angabe, soweit erforderlich, der maßgeblichen Teile | betrifft Anspruch | |
| | AT - B - 304 534 (BYK GULDEN LOMBERG) + Seiten 2-4,6,7 + -- | 1,3-5 | C 07 D 231/12<br>C 07 D 401/12<br>C 07 D 403/06<br>C 07 D 413/06<br>C 07 D 417/06<br>A 61 K 31/415<br>A 61 K 31/425<br>A 61 K 31/445<br>A 61 K 31/495/<br>(C 07 D 401/12<br>C 07 D 231/12<br>C 07 D 211/46)<br>(C 07 D 403/06<br>C 07 D 231/12 |
| | US - A - 3 478 032 (ARYA) + Spalten 2,3,14 + ---- | 1,4,5 | RECHERCHIERTE SACHGEBIETE (Int. ClX 3)<br>C 07 D 231/00<br>C 07 D 401/00<br>C 07 D 403/00<br>C 07 D 413/00<br>C 07 D 417/00<br>A 61 K 31/00 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| WIEN | 22-04-1980 | HERING |

0014847

Europäisches Patentamt

# EUROPÄISCHER RECHERCHENBERICHT

Nummer der Anmeldung

EP 80100294.0

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | | | C 07 D 257/04<br>(C 07 D 413/06<br>C 07 D 231/12<br>C 07 D 263/56)<br>(C 07 D 417/06<br>C 07 D 231/12<br>C 07 D 277/64)<br>(C 07 D 417/06<br>C 07 D 231/06<br>C 07 D 231/12<br>C 07 D 277/06) |
| | | | RECHERCHIERTE SACHGEBIETE (Int. Cl. 3) |
| | | | |

EPA Form 1503.2 06.78